# NOUVEAU FASCICULE DE BREVET EUROPEEN

(11) **EP 0 774 248 B2**
(45) Date de publication et mention de la décision concernant l'opposition: **12.03.2014**
(45) Mention de la délivrance du brevet: 10.02.1999
(21) Numéro de dépôt: 96402248.7
(22) Date de dépôt: 22.10.1996
(51) Int. Cl.: A61K 8/68, A61K 8/73, A61K 8/81, A61K 8/86, A61K 8/898, A61Q 5/06

(54) **Composition pour le traitement des fibres kératiniques comprenant au moins un polymère fixant et au moins un composé de type céramide et procédés**
Zusammensetzung zur Behandlung von Keratinfasern, die mindestens ein fixierendes Polymer und mindestens eine Verbindung vom Ceramidtyp enthält, und Verfahren
Keratinous fiber treating composition containing at least a binding polymer and at least a ceramide-type compound and methods

(30) Priorité: 23.10.1995 FR 9512448
(43) Date de publication de la demande: 21.05.1997
(73) Titulaire: L'Oréal, 75008 Paris (FR)
(72) Inventeur: Dupuis, Christine, 75018 Paris (FR); Dubief, Claude, 78150 Le Chesnay (FR); Cretois, Isabelle, 75012 Paris (FR); Braida-Valerio, Damarys, 75012 Paris (FR)
(74) Mandataire: Dossmann, Gérard

(56) Documents cités:
- EP-A- 0 278 505
- EP-A- 0 455 429
- EP-A- 0 647 617
- EP-A- 0 680 743
- WO-A-92/17160
- DE-T2- 69 100 990
- FR-A- 2 679 770
- FR-A- 2 718 960

## Description

La présente invention a trait à une composition cosmétique pour le traitement des cheveux, comprenant au moins un polymère fixant et au moins un composé de type céramide ainsi qu'au procédé de traitement non-thérapeutique à l'aide de cette composition.

Les compositions de maintien ou de mise en forme des cheveux contenant dans leur formulation des polymères de coiffage (polymères fixants) présentent généralement l'inconvénient de rendre difficile le démêlage, le recoiffage ou le brossage des cheveux, en particulier pendant un brushing.
Pendant le brushing, les cheveux sont abîmés par la chaleur du séchoir et le passage de la brosse dans les cheveux pour mettre en forme la chevelure.
De nombreux cheveux sont ainsi cassés lors d'un brushing. On recherche donc des compositions permettant de protéger les cheveux de cette casse lors de ces agressions.

La mise en oeuvre de dérivés siliconés en association avec des polymères fixants est connue dans la préparation de compositions cosmétiques pour le maintien de la coiffure. Il a été constaté que ces dérivés siliconés améliorent les propriétés de démêlage, de douceur et de brillance des cheveux traités à l'aide de ces compositions. Cependant, d'une part, les dérivés siliconés ne sont pas favorables aux propriétés coiffantes des compositions contenant des polymères fixants et, d'autre part, l'effet de protection vis-à-vis de la casse des cheveux n'est pas encore satisfaisant.

Or, la demanderesse a découvert de manière surprenante qu'en utilisant des compositions contenant un polymère fixant en association avec des composés de type céramide, on obtenait un très bon effet de protection vis-à-vis de la casse des cheveux en particulier pendant un brushing tout en ayant d'excellentes propriétés coiffantes.

Les propriétés coiffantes sont du même niveau ou même supérieures à celles d'une composition ne contenant que le polymère fixant. En particulier, le pouvoir fixant, la tenue dans le temps et le volume de chevelure sont très bons.

Cette découverte est à la base de la présente invention.

L'invention a donc pour objet une composition cosmétique non détergente sous forme de lotion de mises en plis, de lotion pour le brushing, de composition de fixation ou de coiffage des cheveux, caractérisée par le fait qu'elle contient dans un milieu cosmétiquement acceptable au moins un polymère fixant anionique, non ionique, zwittérionique ou amphotère et au moins un composé de type céramide, lesdites compositions ne contenant pas de polymère de vinylpyrrolidone et/ou de polymère cationique comportant des groupements amine primaires, secondaires, tertiaires ou ammonium quaternaire dans la chaîne principale et ayant une viscosité à 1% en poids de matière active dans de l'eau inférieure à 15 mPa.s.

L'invention a encore pour objet l'utilisation de la composition définie ci-dessus pour protéger les cheveux pendant le brushing.

Ces compositions permettent également d'améliorer les propriétés cosmétiques en particulier la douceur et le lissage des cheveux.

Par pouvoir fixant de la composition, on désigne l'aptitude de cette dernière à donner aux cheveux une cohésion telle que la mise en forme initiale de la coiffure est conservée. Par polymère fixant, on entend tout polymère ayant pour fonction de fixer temporairement la forme de la coiffure.

Le terme non détergent signifie que la composition ne permet pas d'éliminer d'un milieu solide tel que par exemple les cheveux, les salissures qui y adhèrent par leur mise en dispersion ou en solution. En particulier, les compositions selon l'invention comprennent moins de 4% en poids par rapport au poids total de la composition de tensioactifs détergents anioniques ou amphotères.

Par polymère de vinylpyrrolidone, on désigne les polymères contenant au moins le monomère vinylpyrrolidone.

Selon la présente invention, on entend, par composé de type céramide, les céramides et/ou les glycocéramides et/ou les pseudocéramides et/ou les néocéramides, naturelles ou synthétiques.

Des composés de type céramides sont par exemple décrits dans les demandes de brevet DE4424530, DE4424533, DE4402929, DE4420736, WO95/23807, WO94/0784 EP-A-0646572, WO95/16665, FR-2 673 179, EP-A-0227994 et WO94/07844, WO94/24097, WO94/10131 dont les enseignements sont ici inclus à titre de référence.

Les composés de type céramide utilisables selon la présente invention répondent préférentiellement à la formule générale (I): 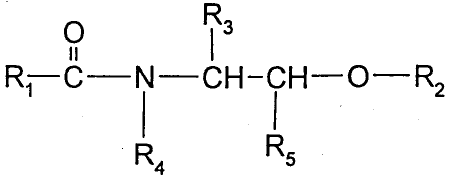 dans laquelle :
- R₁ désigne :
   - soit un radical hydrocarboné, linéaire ou ramifié, saturé ou insaturé, en C₁-C₅₀, de préférence en C₅-C₅₀, ce radical pouvant être substitué par un ou plusieurs groupements hydroxyle éventuellement estérifié par un acide R₇COOH, R₇ étant un radical hydrocarboné, saturé ou insaturé, linéaire ou ramifié, éventuellement mono ou polyhydroxylé, en C₁-C₃₅, le ou les hydroxyles du radical R₇ pouvant être estérifié par un acide gras saturé ou insaturé, linéaire ou ramifié, éventuellement mono ou polyhydroxylé, en C₁ -C₃₅;
   - soit un radical R"-(NR-CO)-R', R désigne un atome d'hydrogène ou un radical hydrocarboné C₁ -C₂₀ mono ou polyhydroxylé, préférentiellement monohydroxylé, R' et R" sont des radicaux hydrocarbonés dont la somme des atomes de carbone est comprise entre 9 et 30, R' étant un radical divalent.
   - soit un radical R₈-O-CO-(CH2)ₚ, R₈ désigne un radical hydrocarboné en C₁-C₂₀, p est un entier variant de 1 à 12.
- R₂ est choisi parmi un atome d'hydrogène, un radical de type saccharidique, en particulier un radical (glycosyle)ₙ, (galactosyle)ₘ ou sulfogalactosyle, un résidu de sulfate ou de phosphate, un radical phosphoryléthylamine et un radical phosphoryléthylammonium, dans lesquels n est un entier variant de 1 à 4 et m est un entier variant de 1 à 8 ;
- R₃ désigne un atome d'hydrogène ou un radical hydrocarboné en C₁ -C₃₃, saturé ou insaturé, hydroxylé ou non, le ou les hydroxyles pouvant être estérifiés par un acide minéral ou un acide R₇COOH, R₇ ayant les mêmes significations que ci-dessus, le ou les hydroxyles pouvant être éthérifiés par un radical (glycosyle)ₙ, (galactosyle)ₘ, sulfogalactosyle, phosphoryléthylamine ou phosphoryléthylammonium, R₃ pouvant également être substitué par un ou plusieurs radicaux alkyle en C₁-C₁₄ ;
   de préférence, R₃ désigne un radical α-hydroxyalkyle en C₁₅-C₂₆, le groupement hydroxyle étant éventuellement estérifié par un α-hydroxyacide en C₁₆-C₃₀ ;
- R₄ désigne un atome d'hydrogène, un radical méthyle, éthyle, un radical hydrocarboné en C₃-C₅₀, saturé ou insaturé, linéaire ou ramifié, éventuellement hydroxylé ou un radical -CH₂-CHOH-CH₂-O-R₆ dans lequel R₆ désigne un radical hydrocarboné en C₁₀-C₂₆ ou un radical R₈-O-CO-(CH₂)ₚ, R₈ désigne un radical hydrocarboné en C₁-C₂₀, p est un entier variant de 1 à 12,
- R₅ désigne un atome d'hydrogène ou un radical hydrocarboné en C₁-C₃₀ saturé ou insaturé, linéaire ou ramifié, éventuellement mono ou polyhydroxylé, le ou les hydroxyles pouvant être éthérifiés par un radical (glycosyle)ₙ, (galactosyle)ₘ, sulfogalactosyle, phosphoryléthylamine ou phosphoryléthylammonium,
   sous réserve que lorsque R₃ et R₅ désignent hydrogène ou lorsque R₃ désigne hydrogène et R₅ désigne méthyle alors R₄ ne désigne pas un atome d'hydrogène, un radical méthyle ou éthyle.

Parmi les composés de formule (I), on préfère les céramides et/ou glycocéramides dont la structure est décrite par DOWNING dans Journal of Lipid Research Vol. 35, 2060-2068, 1994, ou ceux décrits dans la demande de brevet français FR-2 673 179, dont les enseignements sont ici inclus à titre de référence.

Les composés de type céramide plus particulièrement préférés selon l'invention sont les composés de formule (I) pour lesquels R₁ désigne un alkyle saturé ou insaturé dérivé d'acides gras en C₁₄-C₂₂ éventuellement hydroxylé; R₂ désigne un atome d'hydrogène ; et R₃ désigne un radical linéaire en C₁₁₋₁₇ éventuellement hydroxylé et de préférence en C₁₃₋₁₅.

De tels composés sont par exemple :
- le 2-N-linoléoylamino-octadécane-1,3-diol,
- le 2-N-oléoylamino-octadécane-1,3-diol,
- le 2-N-palmitoylamino-octadécane-1,3-diol,
- le 2-N-stéaroylamino-octadécane-1,3-diol,
- le 2-N-béhénoylamino-octadécane-1,3-diol,
- le 2-N-[2-hydroxy-palmitoyl]-amino-octadécane-1,3-diol,
- le 2-N-stéaroyl amino-octadécane-1,3,4 triol et en particulier la N-stéaroyl phytosphingosine,
- le 2-N-palmitoylamino-hexadécane-1,3-diol ou les mélanges de ces composés.

On peut aussi utiliser des mélanges spécifiques tels que par exemple les mélanges de céramide(s) 2 et de céramide(s) 5 selon la classification de DOWNING.

On peut également utiliser les composés de formule (I) pour lesquels R₁ désigne un radical alkyle saturé ou insaturé dérivé d'acides gras en C₁₂-C₂₂ ; R₂ désigne un radical galactosyle ou sulfogalactosyle ; et R₃ désigne un radical hydrocarboné en C₁₂-C₂₂, saturé ou insaturé et de préférence un groupement -CH=CH-(CH₂)₁₂-CH₃.

A titre d'exemple, on peut citer le produit constitué d'un mélange de glycocéramides, vendu sous la dénomination commerciale GLYCOCER par la société WAITAKI INTERNATIONAL BIOSCIENCES.

On peut également utiliser les composés de formule (I) décrits dans les demandes de brevet EP-A-0227994, EP-A-0 647 617, EP-A-0 736 522 et WO 94/07844.

De tels composés sont par exemple le QUESTAMIDE H (bis-(N-hydroxyéthyl N-cétyl) malonamide) vendu par la société OUEST, le N-(2-hydroxyéthyl)-N-(3-cétyloxy-2-hydroxypropyl)amide d'acide cétylique .

On peut également utiliser le N-docosanoyl N-méthyl-D-glucamine décrit dans la demande de brevet WO94/24097.

La concentration en composés de type céramide peut varier entre 0,0001% et 20% en poids environ par rapport au poids total de la composition, et de préférence entre 0,001 et 10% environ et encore plus préférentiellement entre 0,005 et 3 % en poids.

Selon l'invention, on peut utiliser tout polymère fixant connu en soi éliminable au shampooing choisi parmi les polymères anioniques, amphotères, zwittérioniques, non ioniques et leurs mélanges.

Les polymères fixants peuvent être utilisés sous forme solubilisée ou sous forme de latex ou de pseudolatex (dispersion aqueuse de particules insolubles solides de polymère).

Ainsi, les polymères anioniques fixants généralement utilisés sont des polymères comportant des groupements dérivés d'acide carboxylique, sulfonique ou phosphorique et ont un poids moléculaire compris entre environ 500 et 5.000.000.

Ces groupements carboxyliques sont apportés par des monomères mono ou diacides carboxyliques insaturés tels que ceux répondant à la formule : 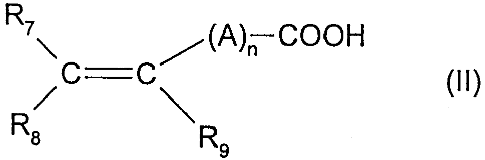 dans laquelle n est un nombre entier de 0 à 10, A désigne un groupement méthylène, éventuellement relié à l'atome de carbone du groupement insaturé ou au groupement méthylène voisin lorsque n est supérieur à 1 par l'intermédiaire d'un hétéroatome tel que oxygène ou soufre, R₇ désigne un atome d'hydrogène, un groupement phényle ou benzyle, R₈ désigne un atome d'hydrogène, un groupement alkyle inférieur ou carboxyle, R₉ désigne un atome d'hydrogène, un groupement alkyle inférieur, un groupement -CH₂ - COOH, phényle ou benzyle.

Dans la formule précitée un radical alkyle inférieur désigne de préférence un groupement ayant 1 à 4 atomes de carbone et en particulier, méthyle et éthyle.

Les polymères anioniques à groupements carboxyliques préférés selon l'invention sont:
A) les homo- ou copolymères d'acide acrylique ou méthacrylique ou leurs sels et en particulier les produits vendus sous les dénominations VERSICOL E ou K par la société ALLIED COLLOID, ULTRAHOLD par la société BASF. Les copolymères d'acide acrylique et d'acrylamide vendus sous la forme de leur sel de sodium sous les dénominations RETEN 421, 423 ou 425 par la Société HERCULES, les sels de sodium des acides polyhydroxycarboxyliques.
B) Les copolymères des acides acrylique ou méthacrylique avec un monomère monoéthylénique tel que l'éthylène, le styrène, les esters vinyliques, les esters d'acide acrylique ou méthacrylique, éventuellement greffés sur un polyalkylène glycol tel que le polyéthylène glycol et éventuellement réticulés. De tels polymères sont décrits en particulier dans le brevet français 1.222.944 et la demande allemande 2.330.956, les copolymères de ce type comportant dans leur chaîne un motif acrylamide éventuellement N-alkylé et/ou hydroxyalkylé tels que décrits notamment dans les demandes de brevets luxembourgeois 75370 et 75371 ou proposés sous la dénomination QUADRAMER par la Société AMERICAN CYANAMID. On peut également citer les copolymères d'acide acrylique et de méthacrylate d'alkyle en C₁-C₄ et le copolymère d'acide méthacrylique et d'acrylate d'éthyle vendu sous la dénomination LUVIMER MAEX par la société BASF.
C) les copolymères dérivés d'acide crotonique tels que ceux comportant dans leur chaîne des motifs acétate ou propionate de vinyle et éventuellement d'autres monomères tels que esters allylique ou méthallylique, éther vinylique ou ester vinylique d'un acide carboxylique saturé linéaire ou ramifié à longue chaîne hydrocarbonée tels que ceux comportant au moins 5 atomes de carbone, ces polymères pouvant éventuellement être greffés et réticulés ou encore un ester vinylique, allylique ou méthallylique d'un acide carboxylique α- ou β-cyclique. De tels polymères sont décrits entre autres dans les brevets français 1.222.944, 1.580.545, 2.265.782, 2.265.781, 1.564.110 et 2.439.798. Des produits commerciaux entrant dans cette classe sont les résines 28-29-30, 26-13-14 et 28-13-10 vendues par la société NATIONAL STARCH.
D) les polymères dérivés d'acides ou d'anhydrides maléique, fumarique, itaconique avec des esters vinyliques, des éthers vinyliques, des halogénures vinyliques, des dérivés phénylvinyliques, l'acide acrylique et ses esters ; ces polymères peuvent être estérifiés. De tels polymères sont décrits en particulier dans les brevets US 2.047.398, 2.723.248, 2.102.113, le brevet GB 839.805 et notamment ceux vendus sous les dénominations GANTREZ AN ou ES par la société ISP.
   Des polymères entrant également dans cette classe sont les copolymères d'anhydrides maléique, citraconique, itaconique et d'un ester allylique ou méthallylique comportant éventuellement un groupement acrylamide, méthacrylamide, une α-oléfine, des esters acryliques ou méthacryliques, des acides acrylique ou méthacrylique dans leur chaîne, les fonctions anhydrides sont monoestérifiées ou monoamidifiées. Ces polymères sont par exemple décrits dans les brevets français 2.350.384 et 2.357.241 de la demanderesse.
E) les polyacrylamides comportant des groupements carboxylates.

Les polymères comprenant les groupements sulfoniques sont des polymères comportant des motifs vinylsulfonique, styrène sulfonique, naphtalène sulfonique ou acrylamido alkylsulfonique.

Ces polymères peuvent être notamment choisis parmi :
- les sels de l'acide polyvinylsulfonique ayant un poids moléculaire compris entre environ 1.000 et 100.000 ainsi que les copolymères avec un comonomère insaturé tel que les acides acrylique ou méthacrylique et leurs esters ainsi que l'acrylamide ou ses dérivés, les éthers vinyliques et la vinylpyrrolidone.
- les sels de l'acide polystyrène sulfonique les sels de sodium ayant un poids moléculaire d'environ 500.000 et d'environ 100.000 vendus respectivement sous les dénominations Flexan 500 et Flexan 130 par National Starch.
   Ces composés sont décrits dans le brevet FR 2.198.719.
- les sels d'acides polyacrylamide sulfoniques ceux mentionnés dans le brevet US 4.128.631 et plus particulièrement l'acide polyacrylamidoéthylpropane sulfonique vendu sous la dénomination COSMEDIA POLYMER HSP 1180 par Henkel.

Selon l'invention, les polymères anioniques sont de préférence choisis parmi les copolymères d'acide acrylique tels que le terpolymère acide acrylique / acrylate d'éthyle / N-tertiobutylacrylamide vendu sous la dénomination ULTRAHOLD STRONG par la société BASF, les copolymères dérivés d'acide crotonique tels que les terpolymères acétate de vinyle / tertio-butyl benzoate de vinyle / acide crotonique et les terpolymères acide crotonique / acétate de vinyle/néododécanoate de vinyle vendus sous la dénomination Résine 28-29-30 par la société NATIONAL STARCH, les polymères dérivés d'acides ou d'anhydrides maléique, fumarique, itaconique avec des esters vinyliques, des éthers vinyliques, des halogénures vinyliques, des dérivés phénylvinyliques, l'acide acrylique et ses esters tels que le copolymère méthylvinyléther/anhydride maléique mono estérifié vendu sous la dénomination GANTREZ ES 425 par la société ISP, les copolymères d'acide méthacrylique et de méthacrylate de méthyle vendus sous la dénomination EUDRAGIT L par la société ROHM PHARMA, le copolymère d'acide méthacrylique et d'acrylate d'éthyle vendu sous la dénomination LUVIMER MAEX par la société BASF et le copolymère acétate de vinyle/acide crotonique vendu sous la dénomination LUVISET CA 66 par la société BASF et le terpolymère acétate de vinyle/acide crotonique/polyéthylèneglycol sous la dénomination ARISTOFLEX A par la société BASF.

Les polymères anioniques les plus particulièrement préférés sont choisis parmi le copolymère méthylvinyléther / anhydride maléique mono estérifié vendu sous la dénomination GANTREZ ES 425 par la société ISP, le terpolymère acide acrylique / acrylate d'éthyle / N-tertiobutylacrylamide vendu sous la dénomination ULTRAHOLD STRONG par la société BASF, les copolymères d'acide méthacrylique et de méthacrylate de méthyle vendus sous la dénomination EUDRAGIT L par la société ROHM PHARMA, les terpolymères acétate de vinyle / tertio-butyl benzoate de vinyle / acide crotonique et les terpolymères acide crotonique / acétate de vinyle / néododécanoate de vinyle vendus sous la dénomination Résine 28-29-30 par la société NATIONAL STARCH, le copolymère d'acide méthacrylique et d'acrylate d'éthyle vendu sous la dénomination LUVIMER MAEX par la société BASF, le terpolymère de vinylpyrrolidone / acide acrylique/méthacrylate de lauryle vendu sous la dénomination ACRYLIDONE LM par la société ISP.

Les polymères amphotères ou zwittérioniques utilisables conformément à l'invention peuvent être choisis parmi les polymères comportant des motifs B et C répartis statistiquement dans la chaîne polymère où B désigne un motif dérivant d'un monomère comportant au moins un atome d'azote basique et C désigne un motif dérivant d'un monomère acide comportant un ou plusieurs groupements carboxyliques ou sulfoniques ou bien B et C peuvent désigner des groupements dérivant de monomères zwittérioniques de carboxybétaïnes ou de sulfobétaïnes;
B et C peuvent également désigner une chaîne polymère cationique comportant des groupements amine primaire, secondaire, tertiaire ou quaternaire, dans laquelle au moins l'un des groupements amine porte un groupement carboxylique ou sulfonique relié par l'intermédiaire d'un radical hydrocarboné ou bien B et C font partie d'une chaîne d'un polymère à motif éthylène α,β-dicarboxylique dont l'un des groupements carboxyliques a été amené à réagir avec une polyamine comportant un ou plusieurs groupements amine primaire ou secondaire.

Les polymères fixants amphotères répondant à la définition donnée ci-dessus plus particulièrement préférés sont choisis parmi les polymères suivants :
1) les polymères résultant de la copolymérisation d'un monomère dérivé d'un composé vinylique portant un groupement carboxylique tel que plus particulièrement l'acide acrylique, l'acide méthacrylique, l'acide maléïque, l'acide alpha-chloracrylique, et d'un monomère basique dérivé d'un composé vinylique substitué contenant au moins un atome basique tel que plus particulièrement les dialkylamino-alkylméthacrylate et acrylate, les dialkylaminoalkylméthacrylamide et acrylamide. De tels composés sont décrits dans le brevet américain n° 3 836 537.
(2) les polymères comportant des motifs dérivant :
   a) d'au moins un monomère choisi parmi les acrylamides ou les méthacrylamides substitués sur l'azote par un radical alkyle,
   b) d'au moins un comonomère acide contenant un ou plusieurs groupements carboxyliques réactifs, et
   c) au moins un comonomère basique tel que des esters à substituants amine primaire, secondaire, tertiaire et
      quaternaire des acides acrylique et méthacrylique et le produit de quaternisation du méthacrylate de diméthylaminoéthyle avec le sulfate de diméthyle ou diéthyle.
   Les acrylamides ou méthacrylamides N-substitués plus particulièrement préférés selon l'invention sont les groupements dont les radicaux alkyle contiennent de 2 à 12 atomes de carbone et plus particulièrement le N-éthylacrylamide, le N-tertiobutyl acrylamide, le N-tertiooctyl acrylamide, le N-octylacrylamide, le N-décylacrylamide, le N-dodécylacrylamide ainsi que les méthacrylamides correspondants.
   Les comonomères acides sont choisis plus particulièrement parmi les acides acrylique, méthacrylique, crotonique, itaconique, maléïque, fumarique ainsi que les monoesters d'alkyle ayant 1 à 4 atome de carbone des acides ou des anhydrides maléïque ou fumarique.
   Les comonomères basiques préférés sont des méthacrylates d'aminoéthyle, de butyl aminoéthyle, de N,N'-diméthylaminoéthyle, de N-tertio-butylaminoéthyle.
   On utilise particulièrement les copolymères dont la dénomination CTFA (4ème Ed., 1991) est Octylacrylamide/acrylates/butylaminoethylmethacrylate copolymer tels que les produits vendus sous la dénomination AMPHOMER ou LOVOCRYL 47 par la société NATIONAL STARCH.
(3) les polyamino amides réticulés et alcoylés partiellement ou totalement dérivant de poyaminoamides de formule générale: 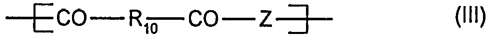 dans laquelle R₁₀ représente un radical divalent dérivé d'un acide dicarboxylique saturé, d'un acide aliphatique mono ou dicarboxylique à double liaison éthylénique, d'un ester d'un alcanol inférieur ayant 1 à 6 atome de carbone de ces acides ou d'un radical dérivant de l'addition de l'un quelconque desdits acides avec une amine bis primaire ou bis secondaire, et Z désigne un radical d'une polyalkylène-polyamine bis-primaire, mono ou bis-secondaire et de préférence représente :
   a) dans les proportions de 60 à 100 moles %, le radical 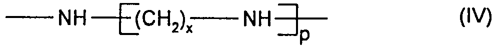 où x=2 et p=2 ou 3, ou bien x=3 et p=2 ce radical dérivant de la diéthylène triamine, de la triéthylène tétraamine ou de la dipropylène triamine;
   b) dans les proportions de 0 à 40 moles % le radical (IV) ci-dessus, dans lequel x=2 et p=1 et qui dérive de l'éthylènediamine, ou le radical dérivant de la pipérazine : 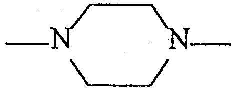
   c) dans les proportions de 0 à 20 moles % le radical -NH-(CH₂)₆-NH-dérivant de l'hexaméthylènediamine, ces polyaminoamines étant réticulées par addition d'un agent réticulant bifonctionnel choisi parmi les épihalohydrines, les diépoxydes, les dianhydrides, les dérivés bis insaturés, au moyen de 0,025 à 0,35 mole d'agent réticulant par groupement amine du polyaminoamide et alcoylés par action d'acide acrylique, d'acide chloracétique ou d'une alcane sultone ou de leurs sels.
   Les acides carboxyliques saturés sont choisis de préférence parmi les acides ayant 6 à 10 atomes de carbone tels que l'acide adipique, triméthyl-2,2,4-adipique et triméthyl-2,4,4-adipique, téréphtalique, les acides à double liaison éthylénique comme par exemple les acides acrylique, méthacrylique, itaconique.
   Les alcanes sultones utilisées dans l'alcoylation sont de préférence la propane ou la butane sultone, les sels des agents d'alcoylation sont de préférence les sels de sodium ou de potassium.
(4) les polymères comportant des motifs zwittérioniques de formule : 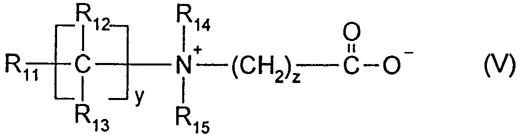 dans laquelle R₁₁ désigne un groupement insaturé polymérisable tel qu'un groupement acrylate, méthacrylate, acrylamide ou méthacrylamide, y et z représente un nombre entier de 1 à 3, R₁₂ et R₁₃ représentent un atome d'hydrogène, méthyle, éthyle ou propyle, R₁₄ et R₁₅ représentent un atome d'hydrogène ou un radical alkyle de telle façon que la somme des atomes de carbone dans R₁₄ et R₁₅ ne dépasse pas 10.
   Les polymères comprenant de telles unités peuvent également comporter des motifs dérivés de monomères non zwittérioniques tels que l'acrylate ou le méthacrylate de diméthyl ou diéthylaminoéthyle ou des alkyle acrylates ou méthacrylates, des acrylamides ou méthacrylamides ou l'acétate de vinyle.
   A titre d'exemple, on peut citer le copolymère de méthacrylate de méthyle / diméthyl carboxyméthylammonio éthylméthacrylate de méthyle tel que le produit vendu sous la dénomination DIAFORMER Z301 par la société SANDOZ.
(5) les polymères dérivés du chitosane comportant des motifs monomères répondant aux formules suivantes : 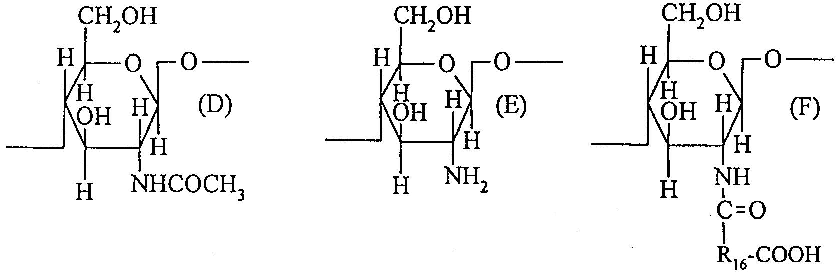 le motif D étant présent dans des proportions comprises entre 0 et 30%, le motif E dans des proportions comprises entre 5 et 50% et le motif F dans des proportions comprises entre 30 et 90%, étant entendu que dans ce motif F, R₁₆ représente un radical de formule : 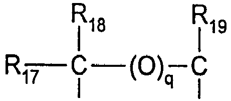 dans laquelle si q=0, R₁₇, R₁₈ et R₁₉, identiques ou différents, représentent chacun un atome d'hydrogène, un reste méthyle, hydroxyle, acétoxy ou amino, un reste monoalcoylamine ou un reste dialcoylamine éventuellement interrompus par un ou plusieurs atomes d'azote et/ou éventuellement substitués par un ou plusieurs groupes amine, hydroxyle, carboxyle, acoylthio, sulfonique, un reste alcoylthio dont le groupe alcoyle porte un reste amino, l'un au moins des radicaux R₁₇, R₁₈ et R₁₉ étant dans ce cas un atome d'hydrogène ;
   ou si q=1, R₁₇, R₁₈ et R₁₉ représentent chacun un atome d'hydrogène, ainsi que les sels formés par ces composés avec des bases ou des acides.
(6) Les polymères dérivés de la N-carboxyalkylation du chitosane comme le N-carboxyméthyl chitosane ou le N-carboxybutyl chitosane vendu sous la dénomination "EVALSAN" par la société JAN DEKKER.
(7) Les polymères répondant à la formule générale (VI) sont décrits dans le brevet français 1 400 366: 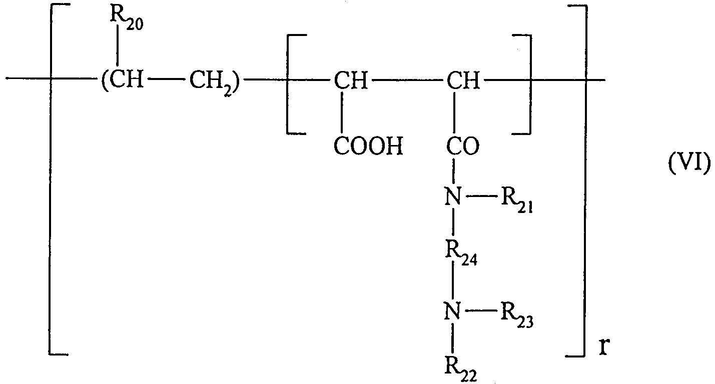 dans laquelle R₂₀ représente un atome d'hydrogène, un radical CH₃O, CH₃CH₂O, phényle, R₂₁ désigne l'hydrogène ou un radical alkyle inférieur tel que méthyle, éthyle, R₂₂ désigne l'hydrogène ou un radical alkyle inférieur tel que méthyle, éthyle, R₂₃ désigne un radical alkyle inférieur tel que méthyle, éthyle ou un radical répondant à la formule : -R₂₄-N(R₂₂)₂, R₂₄ représentant un groupement -CH₂-CH₂- , -CH₂-CH₂-CH₂- , -CH₂-CH(CH₃)- , R₂₂ ayant
   les significations mentionnées ci-dessus,
   ainsi que les homologues supérieurs de ces radicaux et contenant jusqu'à 6 atomes de carbone.
(8) Des polymères amphotères du type -D-X-D-X choisis parmi:
   a) les polymères obtenus par action de l'acide chloracétique ou le chloracétate de sodium sur les composés comportant au moins un motif de formule :

      -D-X-D-X-D- (VII)

      où D désigne un radical 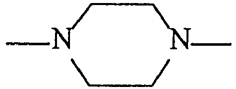 et X désigne le symbole E ou E', E ou E' identiques ou différents désignent un radical bivalent qui est un radical alkylène à chaîne droite ou ramifiée comportant jusqu'à 7 atomes de carbone dans la chaîne principale non substituée ou substituée par des groupements hydroxyle et pouvant comporter en outre des atomes d'oxygène, d'azote, de soufre, 1 à 3 cycles aromatiques et/ou hétérocycliques; les atomes d'oxygène, d'azote et de soufre étant présents sous forme de groupements éther, thioéther, sulfoxyde, sulfone, sulfonium, alkylamine, alkénylamine, des groupements hydroxyle, benzylamine, oxyde d'amine, ammonium quaternaire, amide, imide, alcool, ester et/ou uréthanne.
   b) Les polymères de formule :

      -D-X-D-X- (VII')

      où D désigne un radical 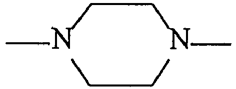 et X désigne le symbole E ou E' et au moins une fois E'; E ayant la signification indiquée ci-dessus et E' est un radical bivalent qui est un radical alkylène à chaîne droite ou ramifiée ayant jusqu'à 7 atomes de carbone dans la chaîne principale, substitué ou non par un ou plusieurs radicaux hydroxyle et comportant un ou plusieurs atomes d'azote, l'atome d'azote étant substitué par une chaîne alkyle interrompue éventuellement par un atome d'oxygène et comportant obligatoirement une ou plusieurs fonction carboxyle ou une ou plusieurs fonctions hydroxyle et bétaïnisées par réaction avec l'acide chloracétique ou du chloracétate de soude.
(9) les copolymères alkyl(C1-C5)vinyléther / anhydride maléique modifié partiellement par semiamidification avec une N,N-dialkylaminoalkylamine telle que la N,N-diméthylaminopropylamine ou par semiestérification avec une N,N-dialcanolamine. Ces copolymères peuvent également comporter d'autres comonomères vinyliques tels que le vinylcaprolactame.

Les polymères amphotères particulièrement préférés selon l'invention sont ceux de la famille (3) tels que les copolymères dont la dénomination CTFA est Octylacrylamide/acrylates/butylaminoethylmethacrylate copolymer tels que les produits vendus sous la dénomination AMPHOMER ou LOVOCRYL 47 par la société NATIONAL STARCH.

Les polymères fixants non ioniques utilisables selon la présente invention sont choisis par exemple parmi :
- les poly β alanines décrites plus particulièrement dans le brevet français n°2508795 ;
- les polyalkyloxazoline telles que les polyéthyloxazoline proposées par la société DOW CHEMICAL sous les dénominations PEOX 50 000, PEOX 200 000 et PEOX 500 000 ;
- les homopolymères d'acétate de vinyle tels que le produit proposé sous le nom de APPRETAN EM par la société HOECHST ou le produit proposé sous le nom de RHODOPAS A 012 par la société RHONE POULENC ;
- les copolymères d'acétate de vinyle et d'ester acrylique tels que le produit proposé sous le nom de RHODOPAS AD 310 de RHONE POULENC ;
- les copolymères d'acétate de vinyle et d'éthylène tels que le produit proposé sous le nom de APPRETAN TV par la société HOECHST ;
- les copolymères d'acétate de vinyle et d'ester maléïque par exemple de maléate de dibutyle tels que le produit proposé sous le nom de APPRETAN MB EXTRA par la société HOECHST ;
- les homopolymères de chlorure de vinyle tels que les produits proposés sous les noms de GEON 460X45, GEON 460X46 et GEON 577 par la société GOODRICH ;
- les cires de polyéthylène tels que les produits proposés sous les dénominations AQUACER 513 et AQUACER 533 par la société BYK CERA ;
- les cires de polyéthylène/polylétrafluoroéthylène tels que les produits proposés sous les dénominations DREWAX D-3750 par la société DREW AMEROID et WAX DISPERSION WD-1077 par la société R.T. NEWEY ;
- les copolymères de polyéthylène et d'anhydride maléïque ;
- les homopolymères d'acrylates d'alkyle et les homopolymères de méthacrylates d'alkyle tels que le produit proposé sous la dénomination MICROPEARL RQ 750 par la société MATSUMOTO ou le produit proposé sous la dénomination LUHYDRAN A 848 S par la société BASF ;
- les copolymères d'esters acryliques tels que par exemple les copolymères d'acrylates d'alkyle et de méthacrylates d'alkyles tels que les produits proposés par la société ROHM&HAAS sous les dénominations PRIMAL ACZ 61 k et EUDRAGIT NE 30 D, par la société BASF sous les dénominations ACRONAL 601, LUHYDRAW LR 8833 ou 8845, par la société HOECHST sous les dénominations APPRETAN N 9213 ou N9212;
- les copolymères d'acrylonitrile et d'un monomère non ionique choisi par exemple parmi le butadiène et les (méth)acrylates d'alkyle ; on peut citer les produits proposés sous les dénominations NIPOL LX 531 B par la société NIPPON ZEON ou ceux proposés sous la dénomination CJ 0601 B par la société ROHM & HAAS
- les homopolymères de styrène tels que le produit RHODOPAS 5051 proposé par la société RHONE POULENC ;
- les copolymères de styrène et de (méth)acrylate d'alkyle tels que les produits MOWILITH LDM 6911, MOWILITH DM 611 et MOWILITH LDM 6070 proposés par la société HOECHST, les produits RHODOPAS SD 215 et RHODOPAS DS 910 proposés par la société RHONE POULENC, le produit URAMUL SC 70 proposé par la société DSM ;
- les copolymères de styrène, de méthacrylate d'alkyle et d'acrylate d'alkyle tels que le produit DAITISOL SPA proposé par la société WACKHERR ;
- les copolymères de styrène et de butadiène tels que les produits RHODOPAS SB 153 et RHODOPAS SB 012 proposés par la société RHONE POULENC ;
- les copolymères de styrène, de butadiène et de vinylpyridine tels que les produits GOODRITE SB VINYLPYRIDINE 2528X10 et GOODRITE SB VINYLPYRIDINE 2508 proposés par la société GOODRICH ;
- les polyuréthannes tels que les produits proposés sous les dénominations ACRYSOL RM 1020 ou ACRYSOL RM 2020 par la société ROHM & HAAS, les produits URAFLEX XP 401 UZ, URAFLEX XP 402 UZ par la société DSM RESINS;
- les copolymères d'acrylate d'alkyle et d'uréthanne tels que le produit 8538-33 par la société NATIONAL STARCH ;
- les polyamides tels que le produit ESTAPOR LO 11 proposé par la société RHONE POULENC.

Les radicaux alkyle des polymères non ioniques ont de préférence de 1 à 6 atomes de carbone.

Selon la présente invention, les polymères fixants sont de préférence des polymères anioniques.

Le ou les polymères fixants sont par exemple présents dans des concentrations comprises entre 0,01 et 20% en poids par rapport au poids total de la composition, de préférence entre 0,1 et 15% en poids et plus particulièrement de 0,5 à 10 % en poids.

Le milieu cosmétiquement ou dermatologiquement acceptable est de préférence constitué par de l'eau ou un mélange d'eau et de solvants cosmétiquement acceptables tels que des monoalcools, des polyalcools, des éthers de glycol ou des esters d'acides gras, qui peuvent être utilisés seuls ou en mélange.

On peut citer plus particulièrement les alcools inférieurs tels que l'éthanol, l'isopropanol, les polyalcools tels que le diéthylèneglycol, les éthers de glycol, les alkyléthers de glycol ou de diéthylèneglycol.

La composition de l'invention peut également contenir au moins un additif choisi parmi les épaississants, les esters d'acides gras, les esters d'acides gras et de glycérol, les silicones, les parfums, les conservateurs, les filtres solaires, les protéines, les vitamines, les polymères, les huiles végétales, animales, minérales ou synthétiques et tout autre additif classiquement utilisé dans le domaine cosmétique. De préférence, la composition contient une silicone telle qu'une huile, une résine, une cire ou une gomme de silicone.

Les compositions selon l'invention peuvent également contenir un ou plusieurs tensioactifs. La nature et la concentration de ces tensioactifs sont choisies par l'homme du métier de façon à ne pas conférer un caractère détergent à la composition.
De préférence, la composition contient moins de 4% en poids de tensioactifs détergents anioniques et/ou amphotères et/ou zwittérioniques.

Ces additifs sont présents dans la composition selon l'invention dans des proportions pouvant aller de 0 à 20% en poids par rapport au poids total de la composition. La quantité précise de chaque additif est fonction de sa nature et est déterminée facilement par l'homme de l'art.

Bien entendu, l'homme de l'art veillera à choisir le ou les éventuels composés à ajouter à la composition selon l'invention de manière telle que les propriétés avantageuses attachées intrinsèquement à la composition conforme à l'invention ne soient pas, ou substantiellement pas, altérées par l'addition envisagée.

En particulier, les compositions selon l'invention comprennent, de préférence, moins de 10 % en poids par rapport au poids total de la composition de corps gras tels que les cires, les huiles, de la paraffine, des esters d'acides gras en C₈-C₃₀. Ainsi, les fibres kératiniques traitées avec les compositions selon l'invention n'ont pas un toucher, ni un aspect gras et le pouvoir fixant de la composition n'est pas diminué. De préférence, la composition selon l'invention ne contient pas ou substantiellement pas de tensioactif cationique.

Les compositions selon l'invention peuvent se présenter sous forme de gel, de lait, de crème, de dispersion, de lotion plus ou moins épaissie ou de mousse.

Les compositions selon l'invention sont utilisées plus particulièrement comme produits non-rincés notamment pour le maintien de la coiffure, la mise en forme ou le coiffage des cheveux.

Les lotions peuvent être conditionnées sous diverses formes notamment dans des vaporisateurs, des flacons pompes ou dans des récipients aérosols afin d'assurer une application de la composition sous forme vaporisée ou sous forme de mousse. De telles formes de conditionnement sont indiquées, par exemple, lorsqu'on souhaite obtenir un spray, une laque ou une mousse pour la fixation ou le traitement des cheveux.

Lorsque la composition selon l'invention est conditionnée sous forme d'aérosol en vue d'obtenir une laque ou une mousse aérosol, elle comprend au moins un agent propulseur qui peut être choisi parmi les hydrocarbures volatils tels que le n-butane, le propane, l'isobutane, le pentane, un hydrocarbure chloré et/ou fluoré et leurs mélanges. On peut également utiliser en tant qu'agent propulseur le gaz carbonique, le protoxyde d'azote, le diméthyléther, l'azote, l'air comprimé et leurs mélanges.

L'invention a encore pour objet un procédé non-thérapeutique de traitement des cheveux humains consistant à appliquer sur celles-ci une composition telle que définie précédemment.

L'invention va être maintenant plus complètement illustrée à l'aide des exemples suivants qui ne sauraient être considérés comme la limitant aux modes de réalisation décrits. (Dans ce qui suit MA signifie Matière Active).

### EXEMPLE 1

On a préparé une lotion de brushing de composition suivante :

| | |
|---|---|
| - N-oléoyldihydrosphingosine (céramide) | 0,02 g |
| - Copolymère méthylvinyléther/anhydride maléique mono estérifié vendu par la société ISP sous la dénomination GANTREZ ES 425 (polymère fixant) | 1 g MA |
| - Ethanol | 50 g |
| - Eau qsp | 100 g |

La composition est préparée au moment de l'emploi en mélangeant une partie A contenant le céramide et 10 g d'éthanol et une partie B contenant le polymère, l'eau et le reste de l'éthanol.

On applique la composition sur les cheveux lavés et essorés, puis on effectue un brushing. Les cheveux séchés sont lisses et doux et présentent de bonnes propriétés coiffantes. Les cheveux résistent bien au brushing.

### EXEMPLE 2

On a préparé une lotion de brushing de composition suivante :

| | |
|---|---|
| - N-oléoyldihydrosphingosine (céramide) | 0,02 g |
| - Copolymère de méthacryloyléthyl N,N-diméthyl carboxyméthylbétaïne et de méthacrylate de butyle vendu en solution à 30% de MA dans l'éthanol sous le nom de DIAFORMER Z301 par la société SANDOZ | 1 gMA |
| - Ethanol | 50 g |
| - Eau déminéralisée qsp | 100 g |

La composition est préparée et appliquée de la même façon qu'à l'exemple 1.
Les cheveux séchés sont lisses et doux et présentent de bonnes propriétés coiffantes.
Les cheveux résistent bien au brushing.

### EXEMPLE 3

On a préparé une lotion de brushing de composition suivante :

| | |
|---|---|
| - N-oléoyldihydrosphingosine (céramide) | 0,02 g |
| - Copolymère méthylvinyléther/anhydride maléique mono estérifié vendu par la société ISP sous la dénomination GANTREZ ES 425 | 1 g MA |
| - Copolymère d'hydroxyéthylcellulose et de chlorure de diallyl diméthyl ammonium vendu sous la dénomination commerciale CELQUAT L200 par la société NATIONAL STARCH | 0,5 g |
| - Ethanol | 50 g |
| - Eau déminéralisée qsp | 100 g |

La composition est préparée et appliquée de la même façon qu'à l'exemple 1.
Les cheveux séchés sont lisses et doux et présentent de bonnes propriétés coiffantes.
Les cheveux résistent bien au brushing.

### EXEMPLE 4

On a préparé une lotion de brushing de composition suivante :

| | |
|---|---|
| - N-oléoyldihydrosphingosine (céramide) | 0,02 g |
| - Terpolymère acétate de vinyle / acide crotonique / polyéthylèneglycol sous la dénomination ARISTOFLEX A par la société BASF | 1 gMA |
| - Amodiméthicone vendu sous la dénomination DC 929 par la société DOW CORNING à 35% de MA | 0,5 gMA |
| - Ethanol | 17,2 g |
| - Eau déminéralisée qsp | 100 g |

La composition est préparée et appliquée de la même façon qu'à l'exemple 1.
Les cheveux séchés sont lisses et doux et présentent de bonnes propriétés coiffantes.
Les cheveux résistent bien au brushing.

### ESSAIS COMPARATIFS

On a préparé quatre compositions 1 A, 2A, 3A et 4A ayant respectivement la même composition que celles des exemples 1, 2, 3 et 4 à l'exception du céramide qui a été supprimé.
On a comparé la masse de cheveux récupérés après un brushing sur perruque pour chaque couple de compositions.

Plus la masse de cheveux cassée est élevée, moins la composition protège les cheveux.

Chaque demi-perruque est préalablement lavée avec 6 ml de shampooing standard. Après rinçage et essorage avec une serviette éponge, on applique 2,4 ml du premier produit sur une demi-perruque à l'aide d'une pipette. On réalise un brushing. Puis on applique 2,4 ml du deuxième produit sur la seconde demi-perruque et on réalise un brushing.
Le brushing est réalisé par un coiffeur expérimenté à l'aide d'une brosse Centaure 3940 et d'un sèche cheveux Mega sprint bi-turbo 1500 (réglage 2 et 2).
Après chaque brushing, les cheveux restés sur la brosse sont récupérés et pesés et l'on compare la masse de cheveux pour chacune des compositions testées.

Les résultats sont rassemblés dans le tableaux ci-dessous :

| **Compositions testées** | **1** Invention | **1A** Comparatif | **2** Invention | **2A** Comparatif | **3** Invention | **3A** Comparatif | **4** Invention | **4A** Comparatif |
|---|---|---|---|---|---|---|---|---|
| **Masse des cheveux récupérés sur la brosse après brushing** | 58,7 | 93,7 | 78,2 | 121,8 | 33,1 | 63,3 | 13,6 | 37,6 |

Pour chaque couple de compositions (1,1 A), (2,2A), (3,3A), (4,4A), on remarque que la masse de cheveux récupérés sur la brosse après le brushing est nettement diminuée pour les compositions selon l'invention 1, 2, 3 et 4 contenant le céramide.

## Revendications

1. Composition cosmétique non détergente sous forme de lotion de mises en plis, de lotion pour le brushing, de composition de fixation ou de coiffage des cheveux, **caractérisée par le fait qu'**elle contient dans un milieu cosmétiquement acceptable au moins un polymère fixant anionique, non ionique, zwittérionique ou amphotère et au moins un composé de type céramides, lesdites compositions ne contenant pas de polymère de vinylpyrrolidone et/ou de polymère cationique comportant des groupements amine primaires, secondaires, tertiaires ou ammonium quaternaire dans la chaîne principale et ayant une viscosité à 1% en poids de matière active dans de l'eau inférieure à 15 mPa.s.

2. Composition selon la revendication 1, **caractérisée par le fait que** le composé de type céramide répond à la formule générale (I): 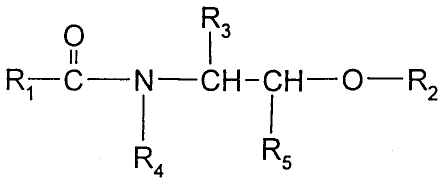 dans laquelle :
- R₁ désigne :
- soit un radical hydrocarboné, linéaire ou ramifié, saturé ou insaturé, en C₁-C₅₀, de préférence en C₅-C₅₀, ce radical pouvant être substitué par un ou plusieurs groupements hydroxyle éventuellement estérifié par un acide R₇COOH, R₇ étant un radical hydrocarboné, saturé ou insaturé, linéaire ou ramifié, éventuellement mono ou polyhydroxylé, en C₁-C₃₅, le ou les hydroxyles du radical R₇ pouvant être estérifié par un acide gras saturé ou insaturé, linéaire ou ramifié, éventuellement mono ou polyhydroxylé, en C₁ -C₃₅;
- soit un radical R"-(NR-CO)-R', R désigne un atome d'hydrogène ou un radical hydrocarboné C₁-C₂₀ mono ou polyhydroxylé, préférentiellement monohydroxylé, R' et R" sont des radicaux hydrocarbonés dont la somme des atomes de carbone est comprise entre 9 et 30, R' étant un radical divalent.
- soit un radical R₈-O-CO-(CH2)ₚ, R₈ désigne un radical hydrocarboné en C₁ -C₂₀, p est un entier variant de 1 à 12.
- R₂ est choisi parmi un atome d'hydrogène, un radical de type saccharidique, en particulier un radical (glycosyle)ₙ, (galactosyle)ₘ ou sulfogalactosyle, un résidu de sulfate ou de phosphate, un radical phosphoryléthylamine et un radical phosphoryléthylammonium, dans lesquels n est un entier variant de 1 à 4 et m est un entier variant de 1 à 8 ;
- R₃ désigne un atome d'hydrogène ou un radical hydrocarboné en C₁ -C₃₃, saturé ou insaturé, hydroxylé ou non, le ou les hydroxyles pouvant être estérifiés par un acide minéral ou un acide R₇COOH, R₇ ayant les mêmes significations que ci-dessus, le ou les hydroxyles pouvant être éthérifiés par un radical (glycosyle)ₙ, (galactosyle)ₘ, sulfogalactosyle, phosphoryléthylamine ou phosphoryléthylammonium, R₃ pouvant également être substitué par un ou plusieurs radicaux alkyle en C₁-C₁₄ ;
de préférence, R₃ désigne un radical a-hydroxyalkyle en C₁₅-C₂₆, le groupement hydroxyle étant éventuellement estérifié par un α-hydroxyacide en C₁₆-C₃₀ ;
- R₄ désigne un atome d'hydrogène, un radical méthyle, éthyle, un radical hydrocarboné en C₃-C₅₀, saturé ou insaturé, linéaire ou ramifié, éventuellement hydroxylé ou un radical -CH₂-CHOH-CH₂-O-R₆ dans lequel R₆ désigne un radical hydrocarboné en C₁₀-C₂₆ ou un radical R₈-O-CO-(CH2)ₚ, R₈ désigne un radical hydrocarboné en C₁-C₂₀, p est un entier variant de 1 à 12,
- R₅ désigne un atome d'hydrogène ou un radical hydrocarboné en C₁-C₃₀ saturé ou insaturé, linéaire ou ramifié, éventuellement mono ou polyhydroxylé, le ou les hydroxyles pouvant être éthérifiés par un radical (glycosyle)ₙ, (galactosyle)ₘ, sulfogalactosyle, phosphoryléthylamine ou phosphoryléthylammonium,
sous réserve que lorsque R₃ et R₅ désignent hydrogène ou lorsque R₃ désigne hydrogène et R₅ désigne méthyle alors R₄ ne désigne pas un atome d'hydrogène, un radical méthyle ou éthyle.

3. Composition selon l'une quelconque des revendications précédentes, **caractérisé en ce que** le composé de type céramide est choisi dans le groupe constitué par:
- le 2-N-linoléoylamino-octadécane-1,3-diol,
- le 2-N-oléoylamino-octadécane-1,3-diol,
- le 2-N-palmitoylamino-octadécane-1,3-diol, 1,
- le 2-N-stéaroylamino-octadécane-1,3-diol,
- le 2-N-béhénoylamino-octadécane-1,3-diol,
- le 2-N-[2-hydroxy-palmitoyl]-amino-octadécane-1,3-diol,
- le 2-N-stéaroyl amino-octadécane-1,3,4 triol,
- le 2-N-palmitoylamino-hexadécane-1,3-diol,
ou les mélanges de ces composés.

4. Composition selon l'une quelconque des revendications 1 ou 2, **caractérisé en ce que** le composé de type céramide est choisi parmi le bis-(N-hydroxyéthyl N-cétyl) malonamide, le N-(2-hydroxyéthyl)-N-(3-cétyloxy-2-hydroxypropyl)amide d'acide cétylique) et le N-docosanoyl N-méthyl-D-glucamine.

5. Composition selon l'une des revendications précédentes, **caractérisée par le fait que** le polymère fixant anionique est choisi parmi :
- les polymères comportant des motifs carboxyliques dérivant de monomères mono ou diacides carboxyliques insaturés de formule : 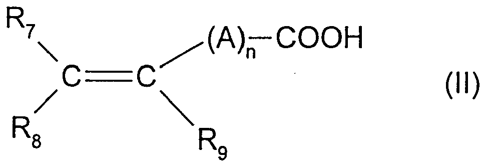 dans laquelle n est un nombre entier de 0 à 10, A désigne un groupement méthylène, éventuellement relié à l'atome de carbone du groupement insaturé ou au groupement méthylène voisin lorsque n est supérieur à 1 par l'intermédiaire d'un hétéroatome tel que oxygène ou soufre, R₇ désigne un atome d'hydrogène, un groupement phényle ou benzyle, R₈ désigne un atome d'hydrogène, un groupement alkyle inférieur ou carboxyle, R₉ désigne un atome d'hydrogène, un groupement alkyle inférieur, un groupement -CH₂-COOH, phényle ou benzyle ;
- les polymères comprenant des motifs dérivant d'acide sulfonique tels que des motifs vinylsulfonique, styrènesulfonique, acrylamido alkylsulfonique.

6. Composition selon la revendication 5, **caractérisée en ce que** le polymère fixant anionique est choisi parmi :
A) les homo- ou copolymères d'acide acrylique ou méthacrylique ou leurs sels, les copolymères d'acide acrylique et d'acrylamide et leurs sels, les sels de sodium d'acides polyhydroxycarboxyliques ;
B) les copolymères des acides acrylique ou méthacryliques avec un monomère monoéthylénique tel que l'éthylène, le styrène, les esters vinyliques, les esters d'acide acrylique ou méthacrylique, éventuellement greffés sur un polyalkylène glycol tel que le polyéthylène glycol et éventuellement réticulés ; les copolymères de ce type comportant dans leur chaîne un motif acrylamide éventuellement N-alkylé et/ou hydroxyalkylé, les copolymères d'acide acrylique et de méthacrylate d'alkyle en C₁-C₄ ;
C) les copolymères dérivés d'acide crotonique tels que ceux comportant dans leur chaîne des motif acétate ou propionate de vinyle et éventuellement d'autres monomères tels que esters allylique ou méthallylique, éther vinylique ou ester vinylique d'un acide carboxylique saturé linéaire ou ramifié à longue chaîne hydrocarbonée tels que ceux comportant au moins 5 atomes de carbone, ces polymères pouvant éventuellement être greffés et réticulés ;
D) les polymères dérivés d'acides ou d'anhydrides maléique, fumarique, itaconique avec des esters vinyliques, des éthers vinyliques, des halogénures vinyliques, des dérivés phénylvinyliques, l'acide acrylique et ses esters ; les copolymères d'anhydrides maléique, citraconique, itaconique et d'un ester allylique ou méthallylique comportant éventuellement un groupement acrylamide, méthacrylamide, une α-oléfine, des esters acryliques ou méthacryliques, des acides acrylique ou méthacrylique dans leur chaîne, les fonctions anhydrides sont monoestérifiées ou monoamidifiées ;
E) les polyacrylamides comportant des groupements carboxylates.

7. Composition selon la revendication 6, **caractérisée en ce que** le polymère fixant anionique est choisi parmi :
- les copolymères d'acide acrylique tels que le terpolymère acide acrylique/acrylate d'éthyle/N-tertiobutylacrylamide;
- les copolymères dérivés d'acide crotonique tels que les terpolymères acétate de vinyle / tertio-butyl benzoate de vinyle / acide crotonique et les terpolymères acide crotonique/acétate de vinyle/néododécanoate de vinyle ;
- les polymères dérivés d'acides ou d'anhydrides maléique, fumarique, itaconique avec des esters vinyliques, des éthers vinyliques, des halogénures vinyliques, des dérivés phénylvinyliques, l'acide acrylique et ses esters tels que les copolymères méthylvinyléther/anhydride maléique mono estérifié ;
- les copolymères d'acide méthacrylique et de méthacrylate de méthyle ;
- le copolymère d'acide méthacrylique et d'acrylate d'éthyle ;
- le copolymère acétate de vinyle/acide crotonique ;
- le terpolymère acétate de vinyle/acide crotonique/polyéthylèneglycol.

8. Composition selon l'une quelconque des revendications 1 à 4, **caractérisée en ce que** le polymère fixant amphotère est choisi parmi les polymères comportant des motifs dérivant:
a) d'au moins un monomère choisi parmi les acrylamides ou les méthacrylamides substitués sur l'azote par un radical alkyle,
b) d'au moins un comonomère acide contenant un ou plusieurs groupements carboxyliques réactifs, et
c) au moins un comonomère basique tel que des esters à substituants amine primaire, secondaire, tertiaire et quaternaire des acides acrylique et méthacrylique et le produit de quaternisation du méthacrylate de diméthylaminoéthyle avec le sulfate de diméthyle ou diéthyle.

9. Composition selon la revendication 8, **caractérisée en ce que** le polymère fixant amphotère est choisi parmi les copolymères dont la dénomination CTFA est Octylacrylamide/ acrylates/ butylaminoethylmethacrylate copolymer.

10. Composition selon l'une quelconque des revendications 1 à 4, **caractérisé par le fait que** le polymère fixant non ionique est choisi parmi :
- les poly β alanines ;
- les polyalkyloxazoline ;
- les homopolymères d'acétate de vinyle ;
- les copolymères d'acétate de vinyle et d'ester acrylique ;
- les copolymères d'acétate de vinyle et d'éthylène ;
- les copolymères d'acétate de vinyle et d'ester maléïque ;
- les homopolymères de chlorure de vinyle ;
- les cires de polyéthylène ;
- les cires de polyéthylène/polylétrafluoroéthylène;
- les copolymères de polyéthylène et d'anhydride maléïque ;
- les homopolymères d'acrylates d'alkyle et les homopolymères de méthacrylates d'alkyle ;
- les copolymères d'esters acryliques tels que par exemple les copolymères d'acrylates d'alkyle et de méthacrylates d'alkyles ;
- les copolymères d'acrylonitrile et d'un monomère non ionique choisi par exemple parmi le butadiène et les (méth)acrylates d'alkyle ;
- les homopolymères de styrène ;
- les copolymères de styrène et de (méth)acrylate d'alkyle ;
- les copolymères de styrène, de méthacrylate d'alkyle et d'acrylate d'alkyle ;
- les copolymères de styrène et de butadiène ;
- les copolymères de styrène, de butadiène et de vinylpyridine ;
- les copolymères d'acrylate d'alkyle et d'uréthanne.

11. Composition selon l'une quelconque des revendications précédentes, **caractérisé par le fait que** le ou les composés de type céramides sont présents dans des concentrations allant de 0,0001 à 20% en poids par rapport au poids total de la composition et de préférence de 0,001 à 10 % en poids et plus préférentiellement entre 0,005 et 3% en poids.

12. Composition selon l'une quelconque des revendications précédentes, **caractérisée par le fait que** le ou les polymères fixants sont utilisés en une quantité allant de 0,01 à 20% en poids par rapport au poids total de la composition, de préférence de 0,1 à 15% en poids et plus particulièrement de 0,5 à 10 % en poids.

13. Composition selon l'une quelconque des revendications précédentes, **caractérisée par le fait qu'**elle contient en plus au moins un additif choisi dans le groupe constitué par les épaississants, les esters d'acides gras, les esters d'acides gras et de glycérol, les silicones, les tensioactifs, les parfums, les conservateurs, les filtres solaires, les protéines, les vitamines, les polymères, les huiles végétales, animales, minérales ou synthétiques et tout autre additif classiquement utilisé dans le domaine cosmétique.

14. Composition selon l'une quelconque des revendications précédentes, **caractérisée par le fait que** le milieu cosmétiquement acceptable est constitué par de l'eau ou un mélange d'eau et d'au moins un solvant cosmétiquement acceptable.

15. Composition selon la revendication 14, **caractérisée par le fait que** les solvants cosmétiquement acceptables sont choisis dans le groupe constitué par les monoalcools, les polyalcools, les éthers de glycol, les esters d'acides gras et leurs mélanges.

16. Composition selon l'une quelconque des revendications précédentes, **caractérisée par le fait qu'**elle se présente sous forme de gel, de lait, de crème, de dispersion, de lotion plus ou moins épaissie ou de mousse.

17. Composition selon l'une quelconque des revendications précédentes, **caractérisée par le fait qu'**elle est un produit de coiffage, de maintien de la coiffure, la mise en forme.

18. Composition selon l'une quelconque des revendications précédentes, **caractérisée par le fait qu'**elle est conditionnée sous forme de vaporisateur, de flacon pompe ou bien dans un récipient aérosol en vue d'obtenir un spray, une laque ou une mousse.

19. Composition selon l'une quelconque des revendications précédentes, **caractérisée par le fait que** le polymère fixant est solubilisé dans le milieu cosmétiquement acceptable ou utilisé sous forme de dispersion aqueuse de particules solides insolubles.

20. Procédé non-thérapeutique de traitement des cheveux, **caractérisée par le fait qu'**il consiste à appliquer sur lesdites matières une composition telle que définie selon l'une quelconque des revendications précédentes.

21. Utilisation d'une composition telle que définie selon l'une quelconque des revendications 1 à 19 pour protéger les cheveux pendant le brushing.

## Patentansprüche

1. Kosmetische, nicht reinigende Zusammensetzung in Form einer Lotion für Wasserwellen, einer Lotion für Fönfrisuren, einer Zusammensetzung zum Fixieren der Haare oder einer Zusammensetzung zum Frisieren der Haare, **dadurch gekennzeichnet, dass** sie in einem kosmetisch akzeptablen Medium mindestens ein anionisches, nichtionisches, zwitterionisches oder amphoteres fixierendes Polymer und mindestens eine Verbindung vom Ceramidtyp enthält, wobei die Zus mensetzungen kein Vinylpyrrolidonpolymer und/oder kein kationisches Polymer mit primären, sekundären oder tertiären Aminogruppen oder quaternäre Ammoniumgruppen in der Hauptkette enthalten und bei einer Menge von 1 Gew.-% wirksamer Substanz in Wasser eine Viskosität unter 15 mPa·s aufweisen.

2. Zusammensetzung nach Anspruch 1, **dadurch gekennzeichnet, dass** die Verbindung vom Ceramidtyp der folgenden Formel (I) entspricht: 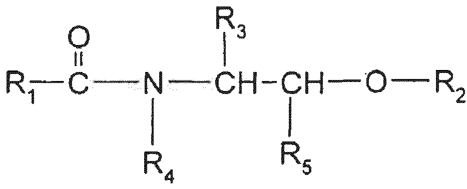 worin bedeuten:
- R₁:
• entweder eine geradkettige oder verzweigte, gesättigte oder ungesättigte C₁₋₅₀-Kohlenwasserstoffgruppe und vorzugsweise C₅₋₅₀-Kohlenwasserstoffgruppe, wobei die Gruppe mit einer oder mehreren Hydroxygruppe substituiert sein kann, die gegebenenfalls mit einer Säure R₇COOH verestert sein können, wobei R₇ eine gesättigte oder ungesättigte, geradkettige oder verzweigte C₁₋₃₅-Kohlenwasserstoffgruppe ist, die gegebenenfalls eine oder mehrere Hydroxygruppen aufweist, wobei die Hydroxygruppe(n) der Gruppe R₇ mit einer gesättigten oder ungesättigten, geradkettigen oder verzweigten, gegebenenfalls einoder mehrfach hydroxylierten C₁₋₃₅-Fettsäure verestert sein kann (können);
• oder eine Gruppe R"-(NR-CO)-R', wobei R ein Wasserstoffatom oder eine C₁₋₂₀-Kohlenwasserstoffgruppe mit einer oder mehreren Hydroxygruppen und vorzugsweise einer Hydroxygruppe bedeutet und R' und R" Kohlenwasserstoffgruppen sind, deren Summe an Kohlenstoffatomen im Bereich von 9 bis 30 liegt, wobei R' eine zweiwertige Gruppe ist;
• oder eine Gruppe R₈-O-CO-(CH₂)ₚ, wobei R₈ eine C₁₋₂₀-Kohlenwasserstoffgruppe ist und p eine ganze Zahl im Bereich von 1 bis 12 bedeutet;
- R₂ eine Gruppe, die ausgewählt ist unter einem Wasserstoffatom, einer Gruppe vom Saccharidtyp, insbesondere (Glycosyl)ₙ, (Galactosyl)ₘ oder Sulfogalactosyl, einer Sulfat- oder Phosphatgruppe, einer Phosphorylethylamingruppe und einer Phosphorylethylammoniumgruppe, wobei n eine ganze Zahl im Bereich von 1 bis 4 und m eine ganze Zahl im Bereich von 1 bis 8 ist;
- R₃ ein Wasserstoffatom oder eine gesättigte oder ungesättigte, gegebenenfalls hydroxylierte C₁₋₃₃-Kohlenwasser-stoffgruppe, wobei die Hydroxygruppe(n) mit einer organischen Säure oder einer Säure R₇COOH verestert sein kahn (können), wobei R₇ die oben angegebenen Bedeutungen aufweist, oder wobei die Hydroxygruppe(n) mit einer (Glycosyl)ₙ-Gruppe, (Galactosyl)ₘ-Gruppe, Sulfogalactosylgruppe, Phosphorylethylamingruppe oder Phosphorylethylammoniumgruppe verethert sein kann (können), und wobei die Gruppe R₃ mit einer oder mehreren C₁₋₁₄-Alkylgruppen substituiert sein kann;
R₃ bedeutet vorzugsweise eine α-Hydroxyalkylgruppe mit 15 bis 26 Kohlenstoffatomen, wobei die Hydroxygruppe gegebenenfalls mit einer α-Hydroxysäure mit 16 bis 30 Kohlenstoffatomen verestert sein kann;
- R₄ ein Wasserstoffatom, Methyl, Ethyl, eine gesättigte oder ungesättigte, geradkettige oder verzweigte, gegebenenfalls hydroxylierte C₃₋₅₀-Kohlenwasserstoffgruppe, eine Gruppe -CH₂-CHOH-CH₂-O-R₆, wobei R₆ eine C₁₀₋₂₆-Kohlenwasserstoffgruppe bedeutet, oder eine Gruppe R₈-O-CO-(CH₂)ₚ, wobei R₈ eine C₁₋₂₀-Kohlenwasserstoffgruppe bedeutet und p eine ganze Zahl im Bereich von 1 bis 12 ist;
- R₅ ein Wasserstoffatom oder eine gesättigte oder ungesättigte, geradkettige oder verzweigte, gegebenenfalls ein- oder mehrfach hydroxylierte C₁₋₃₀-Kohlenwasserstoffgruppe, wobei die Hydroxygruppe(n) mit einer (Glycosyl)ₙ-Gruppe, (Galactosyl)ₘ-Gruppe, Sulfogalactosylgruppe, Phosphorylethylamingruppe oder Phosphorylethylammoniumgruppe verethert sein kann (können);
mit der Maßgabe, dass R₄ nicht Wasserstoff, Methyl oder Ethyl bedeutet, wenn R₃ und R₅ Wasserstoff bedeuten oder R₃ Wasserstoff und R₅ Methyl bedeutet.

3. Zusammensetzung nach einem der vorhergehenden Ansprüche, **dadurch gekennzeichnet, dass** die Verbindung vom Ceramidtyp unter:
- 2-N-Linoleoylamino-octadecan-1,3-diol,
- 2-N-Oleoylamino-octadecan-1,3-diol,
- 2-N-Palmitoylamino-octadecan-1,3-diol,
- 2-N-Stearoylamino-octadecan-1,3-diol,
- 2-N-Behenoylamino-octadecan-1,3-diol
- 2-N-[2-Hydroxy-palmitoyl]-amino-octadecan-1,3-diol,
- 2-N-Stearoylamino-octadecan-1,3,4-triol,
- 2-N-Palmitoylaino-hexadecan-1,3-diol,
oder den Gemischen dieser Verbindungen ausgewählt ist.

4. Zusammensetzung nach einem der Ansprüche 1 oder 2, **dadurch gekennzeichnet, dass** die Verbindung vom Ceramidtyp unter Bis(N-hydroxyethyl-N-cetyl)-malonamid, N-(2-Hydroxyethyl)-N-(3-cetyloxy-2-hydroxypropyl)-cetylsäureamid und N-Docasanoyl-N-methyl-D-glucamin ausgewählt ist.

5. Zusammensetzung nach einem der vorhergehenden Ansprüche **dadurch gekennzeichnet, dass** das anionische fixierende Polymer ausgewählt ist unter:
- den Polymeren mit Carboxygruppen, die von ungesättigten Mono- oder Dicarbonsäuremonomeren der folgenden Formel abgeleitet sind: 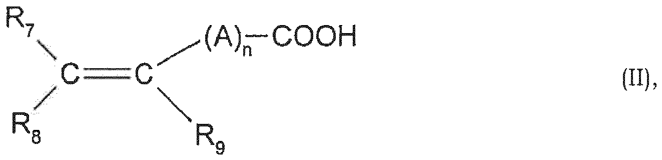 worin n Null oder eine ganze Zahl von 1 bis 10 bedeutet, A eine Methylengruppe bezeichnet, die gegebenenfalls an das Kohlenstoffatom der ungesättigten Gruppe oder, falls n größer 1 ist, an die benachbarte Methylengruppe über ein Heteroatom, wie Sauerstoff oder Schwefel, gebunden ist, R₇ ein Wasserstoffatom, Phenyl oder Benzyl bedeutet, R₈ ein Wasserstoffatom, eine niedere Alkylgruppe oder Carboxy bedeutet und R₉ ein Wasserstoffatom, eine niedere Alkylgruppe, -CH₂-COOH, Phenyl oder Benzyl ist; und
- den Polymeren, die von Sulfonsäuren abgeleitete Einheiten enthalten, wie Vinylsulfonsäure-, Styrolsulfonsäure- und Acrylamidoalkylsulfonsäureeinheiten.

6. Zusammensetzung nach Anspruch 5 **dadurch gekennzeichnet, dass** das anionische polymer ausgewählt ist unter:
A) den Homo- oder Copolymeren von Acrylsäure oder Methacrylsäure oder deren Salzen, den Copolymeren von Acrylsäure und Acrylamid und deren Salzen und den Natriumsalzen von Polyhydroxycarbonsäuren;
B) den Copolymeren von Acrylsäure oder Methacrylsäure mit einem monoethylenisch ungesättigten Monomer, wie Ethylen, Styrol, Vinylestern, Estern von Acrylsäure oder Methacrylsäure, die gegebenenfalls auf ein Polyalkylenglycol, beispielsweise Polyethylenglycol, gepfropft und gegebenenfalls vernetzt sind; den Copolymeren, die in ihrer Kette eine Acryl ideinheit aufweisen, die gegebenenfalls N-alkyliert und/ oder hydroxyalkyliert ist, und den Copolymeren von Acrylsäure und C₁₋₄-Alkylmethacrylat;
C) den Copolymeren, die von Crotonsäure abgeleitet sind, beispielsweise Copolymeren, die in ihrer Kette Vinylacetat- oder VinylpropionatEinheiten und gegebenenfalls weitere Monomere aufweisen, wie Allyloder Methallylester, Vinylether oder Vinylester einer geradkettigen oder verzweigten, gesättigten Carbonsäure mit langer Kohlenwasserstoffkette, wie Carbonsäuren mit mindestens 5 Kohlenstoffatomen, wobei diese Polymere gegebenenfalls gepfropft oder vernetzt sein können;
D) den von Maleinsäure, Fumarsäure, Itaconsäure oder deren Anhydriden abgeleiteten Polymeren mit Vinylestern, Vinylethern, Vinylhalogeniden, Phenylvinylderivaten, Acrylsäure und ihren Estern; und den Copolymeren von Maleinsäure-, Citraconsäure-, Itaconsäureanhydrid mit einem Allylester oder Methallylester, die gegebenenfalls Acrylamid, Methacrylamid, α-Olefin, Acryl- oder Methacrylester, Acryl- oder Methacrylsäure in ihrer Kette enthalten, wobei die Anhydridfunktionen einfach verestert sind oder als einfaches Amid vorliegen;
E) den Polyacrylamide mit Carboxylatgruppen.

7. Zusammensetzung nach Anspruch 6, **dadurch gekennzeichnet, dass** das anionische fixierende Polymer ausgewählt ist unter:
- den Copolymeren von Acrylsäure, wie dem Acrylsäure/Ethylacrylat/N-tert-Butyl-acrylamid-Terpolymer;
- den von Crotonsäure abgeleiteten Copolymeren, wie den Vinylacetat/ Vinyl-tert-butylbenzoat/ Crotonsäure-Terpolymeren und den Crotonsäure/Vinylacetat/Vinylneododecanoat-Terpolymeren,
- den von Maleinsäure, Fumarsäure, Itaconsäure oder deren Anhydriden abgeleiteten Polymeren mit Vinylestern, Vinylethern, Vinylhalogeniden, Phenylvinylderivaten und Acrylsäure und ihren Estern, wie den Methylvinylether / einfach verestertes Maleinsäureanhydrid-Copolymern,
- den Copolymeren von Methacrylsäure und Methylmethacrylat,
- dem Copolymer von Methacrylsäure und Ethylacrylat,
- dem Copolymer von Vinylacetat und Crotonsäure, und
- dem Vinylacetat/Crotonsäure/Polyethylenglycol-Terpolymer.

8. Zusammensetzung nach einem der Ansprüche 1 bis 4, **dadurch gekennzeichnet, dass** das amphotere fixierende Polymer unter den Polymeren ausgewählt ist, die Einheiten enthalten, die abgeleitet sind von:
a) mindestens einem Monomer, das unter den Acrylamiden oder Methyacrylamiden, die am Stickstoffatom mit einer Alkylgruppe substituiert sind, ausgewählt ist,
b) mindestens einem sauren Comonomer, das eine oder mehrere reaktive Carboxygruppen enthält, und
c) mindestens einem basischen Comonomer, wie den Estern von Acrylsäure und Methacrylsäure mit primären, sekundären, tertiären und quaternären Aminogruppen als Substituenten und dem Produkt der Quaternisierung von Dimethylaminoethylmethacrylat mit Dimethyl- oder Diethylsulfat.

9. Zusammensetzung nach Anspruch 8, **dadurch gekennzeichnet, dass** das amphotere fixierende Polymer unter den Copolymeren ausgewählt ist, deren CTFA-Bezeichnung Octylacrylamid/Acrylate/Butylaminoethylmethacrylat-Copolymer ist.

10. Zusammensetzung nach einem der Ansprüche 1 bis 4, **dadurch gekennzeichnet, dass** das nichtionische fixierende Polymer ausgewählt ist unter:
- den Poly-β-alaninen;
- den Polyalkyloxazolinen;
- den Homopolymeren von Vinylacetat;
- den Copolymeren von Vinylacetat und einem Acrylester;
- den Copolymeren von Vinylacetat und Ethylen;
- den Copolymeren von Vinylacetat und einem Maleinsäureester;
- den Homopolymeren von Vinylchlorid;
- den Polyethylenwachsen;
- den Wachsen von Polyethylen und Polytetrafluorethylen;
- den Copolymeren von Polyethylen und Maleinsäureanhydrid;
- den Homopolymeren von Alkylacrylaten und den Homopolymeren von Alkylmethacrylaten;
- den Copolymeren von Acrylestern, wie beispielsweise den Copolymeren Alkylacrylate / Alkylmethacrylate;
- den Copolymeren von Acrylnitril und einem nichtionischen Monomer, das beispielsweise unter Butadien und Alkyl(meth)acrylaten ausgewählt ist;
- den Homopolymeren von Styrol;
- den Copolymeren von Styrol und Alkyl(meth)acrylat;
- den Copolymeren von Styrol, Alkylmethacrylat und Alkylacrylat;
- den Copolymeren von Styrol und Butadien;
- den Copolymeren von Styrol, Butadien und Vinylpyridin;
- den Copolymeren von Alkylacrylat und Urethan.

11. Zusammensetzung nach einem der vorhergehenden Ansprüche, **dadurch gekennzeichnet, dass** die Verbindung(en) vom Ceramidtyp in Konzentrationen im Bereich von 0,0001 bis 20 Gew.-%, bezogen auf das Gesamtgewicht der Zusammensetzung, und vorzugsweise im Bereich von 0,001 bis 10 Gew.-% und noch bevorzugter im Bereich von 0,005 bis 3 Gew.-% vorliegen.

12. Zusammensetzung nach einem der vorhergehenden Ansprüche, **dadurch gekennzeichnet, dass** das fixierende Polymer oder die fixierenden Polymere in einem Mengenanteil im Bereich von 0,01 bis 20 Gew.-%, bezogen auf das Gesamtgewicht der Zusammensetzung, und vorzugsweise im ereich von 0,1 bis 15 Gew.-% und noch bevorzugter im Bereich von 0,5 bis 10 Gew.-% verwendet werden.

13. Zusammensetzung nach einem der vorhergehenden Ansprüche, **dadurch gekennzeichnet, dass** sie ferner mindestens einen Zusatzstoff enthält, der unter den Verdickungsmitteln, Fettsäureestern, Estern von Fettsäuren und Glycerin, Siliconen, grenzflächenaktiven Stoffen, Parfums, Konservierungsmitteln, Sonnenschutzfiltern, Proteinen, Vitaminen, Polymeren, pflanzlichen Ölen, tierischen Ölen, Mineralölen oder synthetischen Ölen oder beliebigen anderen, herkömmlich in der Kosmetik verwendeten Zusatzstoffen ausgewählt ist.

14. Zusammensetzung nach einem der vorhergehenden Ansprüche, **dadurch gekennzeichnet, dass** das kosmetisch akzeptable Medium aus Wasser oder ein Gemisch von Wasser und mindestens einem kosmetisch akzeptablen Lösungsmittel besteht.

15. Zusammensetzung nach Anspruch 14, **dadurch gekennzeichnet, dass** die kosmetisch akzeptablen Lösungsmittel unter Monoalkoholen, Polyalkoholen, Glycolethern, Fettsäureestern und deren Gemischen ausgewählt sind.

16. Zusammensetzung nach einem der vorhergehenden Ansprüche, **dadurch gekennzeichnet, dass** sie als Gel, Milch, Creme, Dispersion, mehr oder weniger dickflüssige Lotion oder Schaum vorliegt.

17. Zusammensetzung nach einem der vorhergehenden Ansprüche, **dadurch gekennzeichnet, dass** sie ein Produkt zum Frisieren, für den Halt der Frisur oder zur Formung darstellt.

18. Zusammensetzung nach einem der vorhergehenden Ansprüche, **dadurch gekennzeichnet, dass** sie in Zerstäubern, Pump-Flakons oder Aerosolbehältern konfektioniert ist, um ein Spray, einen Lack oder einen Schaum zu bilden.

19. Zusammensetzung nach einem der vorhergehenden Ansprüche, **dadurch gekennzeichnet, dass** das fixierende Polymer in einem kosmetisch akzeptablen Medium gelöst ist oder in Form einer wässrigen Dispersion von unlöslichen, festen Partikeln verwendet wird.

20. Nicht therapeutisches Verfähren zur Behandlung von Haaren, das darin besteht, auf die Haare eine Zusammensetzung nach einem der vorhergehenden Ansprüche aufzutragen.

21. Verwendung einer Zusammensetzung nach einem der Ansprüche 1 bis 19 zum Schutz der Haare beim Fönen.

## Claims

1. Non-detergent cosmetic composition in the form of styling lotion, hair drying lotion, or hair setting or hair styling agent, **characterised in that** it contains, in a cosmetically acceptable medium, at least one anionic, nonionic, zwitterionic or amphoteric fixing polymer and at least one compound of the ceramide type, the said compositions not containing any vinylpyrrolidone polymer and/or cationic polymer containing primary, secondary or tertiary amine or quaternary ammonium groups in the principal chain and having a viscosity at 1 % by weight of active substance in water of less than 15 mPa.s.

2. Composition according to claim 1, **characterised in that** the compound of the ceramide type corresponds to the general formula (I): 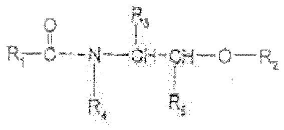 in which:
- R₁ denotes:
- either a saturated or unsaturated, linear or branched, C₁-C₅₀, preferably C₅-C₅₀, hydrocarbon radical, it being possible for this radical to be substituted with one or more hydroxyl groups optionally esterified by an acid R₇COOH, R₇ being an optionally mono- or polyhydroxylated, linear or branched, saturated or unsaturated, C₁-C₃₅ hydrocarbon radical, it being possible for the hydroxyl(s) of the R₇ radical to be esterified by an optionally mono- or polyhydroxylated, linear or branched, saturated or unsaturated, C₁-C₃₅ fatty acid;
- or a radical R"-(NR-CO)-R', R denotes a hydrogen atom or a mono or polyhydroxylated, preferably monohydroxylated, C₁-C₂₀ hydrocarbon radical, R' and R" are hydrocarbon radicals of which the sum of the carbon atoms is between 9 and 30, R' being a divalent radical;
- or a radical R₈-O-CO-(CH2)ₚ, R₈ denotes a C₁-C₂₀ hydrocarbon radical, p is an integer varying from 1 to 12;
- R₂ is chosen from a hydrogen atom, a saccharide-type radical, in particular a (glycosyl)ₙ, (galactosyl)ₘ or sulphogalactosyl radical, a sulphate or phosphate residue, a
phosphorylethylamine radical and a phosphorylethylammonium radical, in which n is an integer varying from 1 to 4 and m is an integer varying from 1 to 8;
- R₃ denotes a hydrogen atom or a hydroxylated or nonhydroxylated, saturated or unsaturated, C₁-C₃₃ hydrocarbon radical, it being possible for the hydroxyl(s) to be esterified by an inorganic acid or an acid R₇COOH, R₇ having the same meanings as above, it being possible for the hydroxyl(s) to be etherified by a (glycosyl)ₙ, (galactosyl)ₘ or sulphogalactosyl radical, or a phosphorylethylamine or phosphorylethylammonium radical, it being also possible for R₃ to be substituted with one or more C₁-C₁₄ alkyl radicals; preferably, R₃ denotes a C₁₅-C₂₆ a-hydroxyalkyl radical, the hydroxyl group being optionally esterified by a C₁₆-C₃₀ α-hydroxy acid;
- R₄ denotes a hydrogen atom, or a methyl or ethyl radical, or an optionally hydroxylated, linear or branched, saturated or unsaturated, C₃-C₅₀ hydrocarbon radical or a radical -CH₂-CHOH-CH₂-O-R₆ in which R₆ denotes a C₁₀-C₂₆ hydrocarbon radical or a radical R₈-O-CO-(CH2)ₚ, where R₈ denotes a C₁-C₂₀ hydrocarbon radical and p is an integer varying from 1 to 12,
- R₅ denotes a hydrogen atom or an optionally mono- or polyhydroxylated, linear or branched, saturated or unsaturated, C₁-C₃₀ hydrocarbon radical, it being possible for the hydroxyl(s) to be etherified by a (glycosyl)ₙ, (galactosyl)ₘ or sulphogalactosyl radical or a phosphorylethylamine or phosphorylethylammonium radical; with the proviso that when R₃ and R₅ denote hydrogen or when R₃ denotes hydrogen and R₅ denotes methyl, then R₄ does not denote a hydrogen atom, or a methyl or ethyl radical.

3. Composition according to any one of the preceding claims, **characterised in that** the compound of the ceramide type is chosen from the group consisting of:
- 2-(N-linoleoylamino)-1,3-octadecanediol,
- 2-(N-oleoylamino)-1,3-octadecanediol,
- 2-(N-palmitoylamino)-1,3-octadecanediol,
- 2-(N-stearoylamino)-1,3-octadecanediol,
- 2-(N-behenoylamino)-1,3-octadecanediol,
- 2-[N-(2-hydroxypalmitoyl)amino]-1,3-octadecanediol,
- 2-(N-stearoylamino)-1,3,4-octadecanetriol,
- 2-(N-palmitoylamino)-1,3-hexadecanediol
or mixtures of these compounds.

4. Composition according to either of claims 1 and 2, **characterised in that** the compound of the ceramide type is chosen from bis-(N-hydroxyethyl-N-cetyl)malonamide, N-(2-hydroxyethyl)-N-(3-cetyloxy-2-hydroxypropyl)amide of cetylic acid and N-docosanoyl-N-methyl-D-glucamine.

5. Composition according to one of the preceding claims, **characterised in that** the anionic fixing polymer is chosen from:
- the polymers containing carboxyl units derived from unsaturated mono- or dicarboxylic acid monomers of formula: 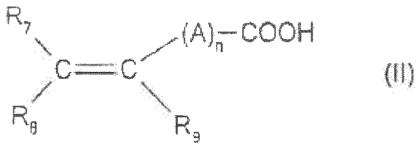 in which n is an integer from 0 to 10, A denotes a methylene group, optionally linked to the carbon atom of the unsaturated group or to the neighbouring methylene group, when n is greater than 1, through a heteroatom such as oxygen or sulphur, R₇ denotes a hydrogen atom, or a phenyl or benzyl group, R₈ denotes a hydrogen atom, or a carboxyl or lower alkyl group, R₉ denotes a hydrogen atom or a lower alkyl group, a group -CH₂-COOH, or a phenyl or benzyl group;
- the polymers comprising units derived from sulphonic acid such as vinylsulphonic, styrenesulphonic, acrylamidoalkylsulphonic units.

6. Composition according to claim 5, **characterised in that** the anionic fixing polymer is chosen from:
A) the homo- or copolymers of acrylic or methacrylic acid or their salts, the copolymers of acrylic acid and of acrylamide and their salts, the sodium salts of polyhydroxycarboxylic acids;
B) the copolymers of acrylic or methacrylic acids with a monoethylene monomer such as ethylene, styrene, vinyl esters, acrylic or methacrylic acid esters, optionally grafted onto a polyalkyleneglycol such as polyethyleneglycol and optionally crosslinked; copolymers of this type containing in their chain an acrylamide unit optionally N-alkylated and/or hydroxyalkylated, the copolymers of acrylic acid and of C₁-C₄ alkylmethacrylate;
C) the copolymers derived from crotonic acid such as those containing in their chain vinyl propionate or acetate units and optionally other monomers such as methallyl or allyl esters, vinyl ether or vinyl ester of a linear or branched saturated carboxylic acid with a long hydrocarbon chain such as those containing at least 5 carbon atoms, it being possible for these polymers to be optionally grafted and crosslinked;
D) the polymers derived from itaconic, fumaric or maleic acids or anhydrides with vinyl esters, vinyl ethers, vinyl halides, phenylvinyl derivatives, acrylic acid and its esters; copolymers of maleic, citraconic and itaconic anhydrides and of an allyl or methallyl ester optionally containing an acrylamide or methacrylamide group, an a-olefin, acrylic or methacrylic esters, acrylic or methacrylic acids in their chain, the anhydride functional groups are monoesterified or monoamidated;
E) the polyacrylamides containing carboxylate groups.

7. Composition according to claim 6, **characterised in that** the anionic fixing polymer is chosen from:
- the acrylic acid copolymers such as the terpolymer acrylic acid/ethyl acrylate/N-tert-butylacrylamide;
- the copolymers derived from crotonic acid such as the terpolymers vinyl acetate/vinyl tert-butylbenzoate/crotonic acid and the terpolymers crotonic acid/vinyl acetate/vinyl neododecanoate;
- the polymers derived from itaconic, fumaric and maleic acids or anhydrides with vinyl esters, vinyl ethers, vinyl halides, phenylvinyl derivatives, acrylic acid and its esters such as the monoesterified maleic anhydride/methylvinyl ether copolymers;
- the copolymers of methacrylic acid and of methylmethacrylate;
- the copolymer of methacrylic acid and of ethyl acrylate;
- the copolymer vinyl acetate/crotonic acid;
- the terpolymer vinyl acetate/crotonic acid/polyethyleneglycol.

8. Composition according to any one of claims 1 to 4, **characterised in that** the amphoteric fixing polymer is chosen from the polymers containing units derived from:
a) at least one monomer chosen from acrylamides or methacrylamides substituted on the nitrogen by an alkyl radical,
b) at least one acidic comonomer containing one or more reactive carboxyl groups, and
c) at least one basic comonomer such as esters with primary, secondary, tertiary and quaternary amine substituents of acrylic and methacrylic acids and the product of quaternization of dimethylaminoethyl methacrylate with dimethyl or diethyl sulphate.

9. Composition according to claim 8, **characterised in that** the amphoteric fixing polymer is chosen from the copolymers whose CTFA name is
Octylacrylamide/acrylates/butylaminoethylmethacrylate copolymer.

10. Composition according to any one of claims 1 to 4, **characterised in that** the nonionic fixing polymer is chosen from:
- the poly-ß-alanines ;
- the polyalkyloxazolines;
- the vinyl acetate homopolymers;
- the acrylic ester and vinyl acetate copolymers;
- the ethylene and vinyl acetate copolymers;
- the copolymers of vinyl acetate and maleic ester;
- the vinyl chloride homopolymers;
- the polyethylene waxes;
- the polyethylene/polytetrafluoroethylene waxes;
- the maleic anhydride and polyethylene copolymers;
- the homopolymers of alkyl acrylates and the homopolymers of alkyl methacrylates;
- the copolymers of acrylic esters such as for example the copolymers of alkyl acrylates and alkyl methacrylates; the copolymers of acrylonitrile and of a nonionic monomer chosen for example from butadiene and alkyl (meth)acrylates;
- the styrene homopolymers;
- the copolymers of styrene and of alkyl (meth)acrylate;
- the copolymers of styrene, alkyl methacrylate and alkyl acrylate;
- the copolymers of styrene and butadiene;
- the copolymers of styrene, butadiene and vinylpyridine;
- the copolymers of alkyl acrylate and urethane.

11. Composition according to any one of the preceding claims, **characterised in that** the compound(s) of the ceramide type is (are) present in concentrations ranging from 0.0001 to 20 % by weight relative to the total weight of the composition and preferably from 0.001 to 10 % by weight and more preferably between 0.005 and 3 % by weight.

12. Composition according to any one of the preceding claims, **characterised in that** the fixing polymer(s) is(are) used in a quantity ranging from 0.01 to 20 % by weight relative to the total weight of the composition, preferably from 0.1 to 15 % by weight and more particularly 0.5 to 10 % by weight.

13. Composition according to any one of the preceding claims, **characterised in that** it contains, in addition, at least one additive chosen from the group consisting of thickeners, fatty acid esters, fatty acid esters and glycerol, silicones, surfactants, perfumes, preservatives, sunscreens, proteins, vitamins, polymers, vegetable, animal, mineral or synthetic oils and any other additive conventionally used in the cosmetic field.

14. Composition according to any one of the preceding claims, **characterised in that** the cosmetically acceptable medium consists of water or a mixture of water and at least one cosmetically acceptable solvent.

15. Composition according to claim 14, **characterised in that** the cosmetically acceptable solvents are chosen from the group consisting of monoalcohols, polyalcohols, glycol ethers, fatty acid esters and mixtures thereof.

16. Composition according to any one of the preceding claims, **characterised in that** it is provided in the form of a gel, milk, cream, dispersion, lotion, which is thickened to a greater or lesser extent, or foam.

17. Composition according to any one of the preceding claims, **characterised in that** it is a product for hair-styling, holding the hair-style and shaping.

18. Composition according to any one of the preceding claims, **characterised in that** it is packaged in the form of a vaporizer, pump dispenser or alternatively in an aerosol container in order to obtain a spray, a lacquer or a foam.

19. Composition according to any one of the preceding claims, **characterised in that** the fixing polymer is solubilized in the cosmetically acceptable medium or used in the form of an aqueous dispersion of insoluble solid particles.

20. Nontherapeutic method of treating hair, **characterised in that** it consists in applying to the said materials a composition as defined according to any one of the preceding claims.

21. Use of a composition as defined according to any one of claims 1 to 19 for protecting the hair during blow-drying.
